(19) Europäisches Patentamt
European Patent Office
Office européen des brevets

(11) Veröffentlichungsnummer: **0 160 818 A1**

(12) # EUROPÄISCHE PATENTANMELDUNG

(21) Anmeldenummer: 85103386.0

(22) Anmeldetag: 22.03.85

(51) Int. Cl.[4]: C 07 D 277/56
C 07 D 277/40

(30) Priorität: 03.04.84 DE 3412293

(43) Veröffentlichungstag der Anmeldung:
13.11.85 Patentblatt 85/46

(84) Benannte Vertragsstaaten:
CH DE FR GB IT LI

(71) Anmelder: BASF Aktiengesellschaft
Carl-Bosch-Strasse 38
D-6700 Ludwigshafen(DE)

(72) Erfinder: Hagen, Helmut, Dr.
Max-Slevogt-Strasse 173
D-6710 Frankenthal(DE)

(72) Erfinder: Ziegler, Hans, Dr.
Am Speyerweg 48
D-6704 Mutterstadt(DE)

(72) Erfinder: Hansen, Guenter, Dr.
Alwin-Mittasch-Platz 8
D-6700 Ludwigshafen(DE)

(54) **Thiazolderivate.**

(57) Die Erfindung betrifft Verbindungen der allgemeinen Formel I

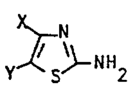

(I)

in der

X Formyl, Carboxyl oder ein Derivat einer Carboxylgruppe und

Y Wasserstoff, Fluor, Chlor, Brom, Nitro, Rhodan, Alkyl- oder Arylsulfonyl oder gegebenenfalls substituiertes Amino, Hydroxy oder Mercapto sind.

Die erfindungsgemäßen Verbindungen eignen sich z.B. als Diazokomponenten zur Herstellung von Farbstoffen.

EP 0 160 818 A1

Croydon Printing Company Ltd.

## Thiazolderivate

Die Erfindung betrifft Verbindungen der allgemeinen Formel I

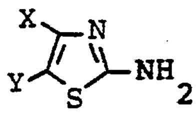 I

in der

X Formyl, Carboxyl oder ein Derivat einer Carboxylgruppe und

Y Wasserstoff, Fluor, Chlor, Brom, Nitro, Rhodan, Alkyl- oder Arylsulfonyl oder gegebenenfalls substituiertes Amino, Hydroxy oder Mercapto sind.

Als Derivate einer Carboxylgruppe sind z.B. Carbonester, Carbonamide, Amidine, Amidoxine, Thioamide und vorzugsweise Cyan zu nennen.

Einzelne Reste X sind neben den bereits genannten z.B. $COOCH_3$, $COOC_2H_5$, $COOC_3H_7$, $COOC_4H_9$, $COOC_6H_{13}$, $COOC_8H_{17}$, $CONH_2$, $CONHCH_3$, $CONHC_2H_5$, $CONHC_3H_7$, $CONHC_4H_9$, $CONHC_6H_{13}$, $CONHC_8H_{17}$,

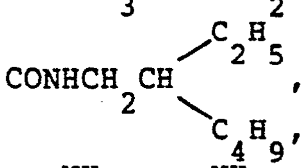 , $CON(CH_3)_2$, $CON(C_4H_9)_2$, 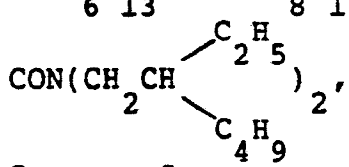

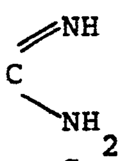 , 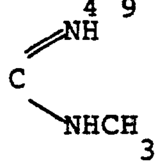 , 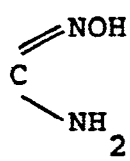 , 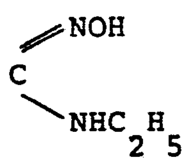 , 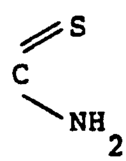 , 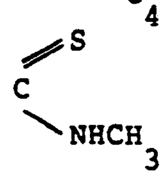 oder

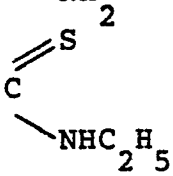 .

Wenn die Alkylreste in n- und i-Form auftreten können, sind beide Isomeren durch die zusammenfassende Formelschreibweise umfaßt.

Bg/P

Reste Y sind neben den bereits genannten z.B. $CH_3SO_2$, $H_5C_2SO_2$, $H_9C_4SO_2$, $H_5C_6SO_2$, $CH_3H_4C_6SO_2$, $NH_2$, $NHCH_3$, $NHC_2H_5$, $NHC_3H_7$, $NHC_4H_9$, $NHC_6H_{13}$, $NHC_8H_{17}$, $OCH_3$, $OC_2H_5$, $OC_3H_7$, $OC_4H_9$, $SCH_3$, $SC_2H_5$, $SC_3H_7$, $SC_4H_9$, $SC_8H_{17}$ oder $SC_{12}H_{25}$ sowie $OC_6H_5$, $OC_6H_4CH_3$, $OC_6H_4OCH_3$, $OC_6H_4Cl$, $SC_6H_5$, $SC_6H_4CH_3$, $SC_6H_4Cl$,

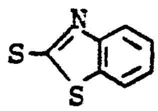 oder 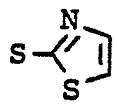 .

Zur Herstellung der Verbindungen der Formel I kann man die Verbindung der Formel

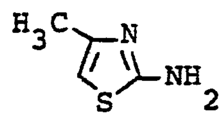

in eine Verbindung der Formel

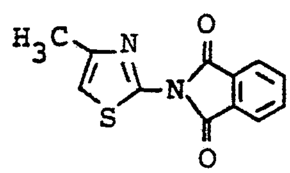

überführen und diese mit Chlor oder Brom zu Verbindungen der Formel

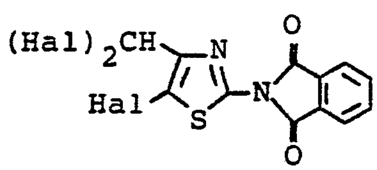

halogenieren. Durch Hydrolyse und gegebenenfalls Oxidation und Austausch erhält man daraus nach an sich bekannten Methoden die Verbindungen der Formel I. Einzelheiten der Herstellung sind in repräsentativen Beispielen beschrieben, in denen sich Angaben über Teile und Prozente, sofern nicht anders vermerkt, auf das Gewicht beziehen.

Die Abspaltung der Phthalimidschutzgruppe wird zweckmäßigerweise nach der in der Patentanmeldung P 33 19 650.8 beschriebenen Methode vorgenommen.

Die Verbindungen der Formel I sind diazotierbar und eignen sich daher als Diazokomponente zur Herstellung von Azofarbstoffen.

Von besonderer Bedeutung sind Verbindungen der Formel Ia

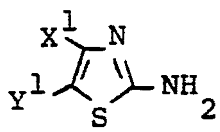

Ia,

in der

$X^1$ CHO, $CH(CO_2CH_3)_2$ oder CN und

$Y^1$ Chlor oder gegebenenfalls substituiertes Amino, Phenylmercapto oder Phenylsulfonyl sind.

Besonders bevorzugt für $X^1$ ist Cyan.

## Beispiele

### Beispiel 1

5-Chlor-4-dichlormethyl-2-phthalimido-thiazol

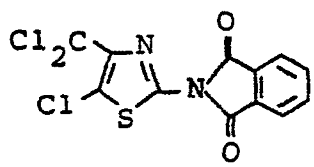

342 Teile 2-Amino-4-methylthiazol wurden mit 4000 Teilen 1,2-Dichlorbenzol und 444 Teilen Phthalsäureanhydrid 1 Stunde auf 160 °C erwärmt. Die Mischung wurde auf 140 °C abgekühlt und mit 2,5 Teilen 6,2'-Azoisobuttersäurenitril versetzt. Bei 140 °C wurden nun 650 Teile Chlor eingeleitet. Die Reaktionsmischung wurde im Vakuum eingeengt, der Niederschlag abgetrennt, mit Petrolether gewaschen und getrocknet.

Es wurden 950 Teile (entsprechend 91 % der Theorie) 5-Chlor-4-dichlormethyl-2-phthalimido-thiazol erhalten.
Schmp.: 166 °C

### Beispiel 2

5-Chlor-4-formyl-2-phthalimido-thiazol-oxim

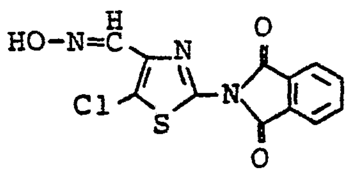

3000 Teile Ameisensäure wurden mit 400 Teilen 5-Chlor-4-dichlormethyl-2-phthalimido-thiazol, 79,9 Teilen Hydroxylammoniumchlorid und 234,6 Teilen Natriumformiat 1 Stunde auf 100 °C erwärmt. Die Reaktionsmischung wurde in 5000 Teile Wasser gegeben, der Niederschlag abgetrennt und getrocknet.

Es wurden 320 Teile (entsprechend 90 % der Theorie) 5-Chlor-4-formyl-2-phthalimido-thiazol-oxim erhalten.
Schmp.: 198 °C

Beispiel 3

5-Chlor-4-cyano-2-phthalimido-thiazol

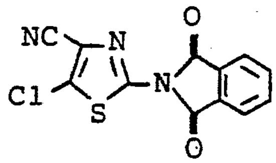

579 Teile 5-Chlor-4-formyl-2-phthalimido-thiazol-oxim wurden in 2000 Teilen Essigsäureanhydrid 12 Stunden auf 120 °C erwärmt. Der Niederschlag wurde abgetrennt und getrocknet. Es wurden 560 Teile (entsprechend 97 % der Theorie) 5-Chlor-4-cyano-2-phthalimido-thiazol erhalten.
Schmp.: 240 °C

Beispiel 4

5-Chlor-4-diacetylmethyl-2-phthalimido-thiazol

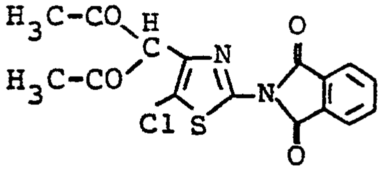

347,5 Teile 5-Chlor-4-dichlormethyl-2-phthalimido-thiazol wurden in 1200 Teilen 50 %iger Schwefelsäure unter Stickstoff 3 Stunden auf 120 °C erwärmt. Der Niederschlag wurde abgetrennt, mit Wasser gewaschen, getrocknet und in 1000 Teilen Essigsäureanhydrid suspendiert. Die Reaktionsmischung wurde 1 Stunde auf 100 °C erwärmt. Der Niederschlag wurde abgetrennt, mit Aceton gewaschen und getrocknet.

Es wurden 340 Teile 5-Chlor-4-diacetylmethyl-2-phthalimido-thiazol (entsprechend 86 % der Theorie) erhalten.
Schmp.: 166 °C

Beispiel 5

2-Amino-5-chlor-4-formyl-thiazol-oxim

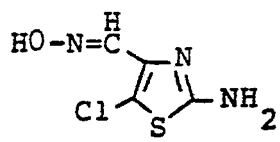

153 Teile 5-Chlor-4-formyl-2-phthalimido-thiazol-oxim wurden mit 40 Teilen Ethanolamin in 1200 Teilen Ethanol 10 Minuten auf 80 °C erwärmt. Die Lösung wurde in Wasser gegeben, der Niederschlag abgetrennt und getrocknet. Es wurden 75 Teile (entsprechend 84 % der Theorie) 2-Amino-5-chlor-4-formyl-thiazol-oxim erhalten.
Schmp.: 194 °C

Beispiel 6

2-Amino-5-chlor-4-cyano-thiazol

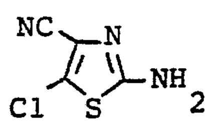

460 Teile 5-Chlor-4-cyano-2-phthalimido-thiazol wurden mit 115 Teilen Ethanolamin in 2000 Teilen Ethanol 5 Minuten auf 80 °C erwärmt. Die Lösung wurde auf Wasser gegeben, der Niederschlag abgesaugt und getrocknet. Es wurden 235 Teile (entsprechend 93 % der Theorie) 2-Amino-5-chlor-4-cyano-thiazol erhalten.
Schmp.: 183 °C

Beispiel 7

2-Amino-5-chlor-4-diacetylmethyl-thiazol

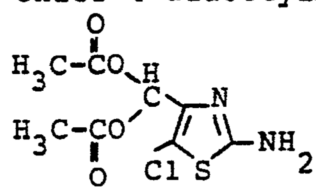

394 Teile 5-Chlor-4-diacetylmethyl-2-phthalimido-thiazol in 4000 Teilen Ethanol wurden bei 20 - 30 °C mit 73 Teilen Ethanolamin versetzt. Die Reaktionsmischung wurde 1 Stunde bei 30 °C gerührt und anschließend auf Wasser gegeben. Der Niederschlag wurde abgetrennt und getrocknet. Es wurden 251 Teile (entsprechend 95 % der Theorie) 2-Amino-5-chlor-4-diacetylmethyl-thiazol erhalten.
Schmp.: 168 °C

Beispiel 8

2-Amino-5-chlor-4-formyl-thiazol

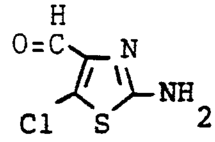

264,5 Teile 2-Amino-5-chlor-4-diacetylmethyl-thiazol wurden in 2000 Teilen 2 n HCl 5 Minuten auf 100 °C erwärmt. Es wurde mit konzentriertem Ammoniak neutralisiert, der Niederschlag abgetrennt und getrocknet. Man erhielt 160 Teile (entsprechend 98 % der Theorie) 2-Amino-5-chlor-4-formyl-thiazol.
Schmp.: 200 °C

Beispiel 9

2-Amino-5-chlor-thiazol-4-carbonsäure

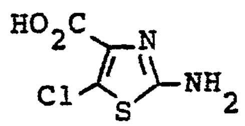

39,2 Teile 2-Amino-5-chlor-4-cyano-thiazol-hydrochlorid wurden in 200 Teilen 50 %iger Schwefelsäure 2 Stunden auf 120 °C erwärmt. Die Reaktionmischung wurde auf Wasser gegeben, der Niederschlag abgetrennt und in verdünntem Ammoniak gelöst. Durch Neutralisation der Lösung wurde die 2-Amino-5-chlor-thiazol-4-carbonsäure ausgefällt. Es wurden 31 Teile (entsprechend 87 % der Theorie) erhalten.
Schmp.: 225 °C

Beispiel 10

2-Amino-4-cyano-5-diethylamino-thiazol

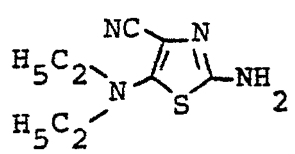

32 Teile 2-Amino-5-chlor-4-cyano-thiazol wurden in 400 Teilen Methylglykol mit 44 Teilen Diethylamin auf 110 °C erwärmt. Nach Abkühlen wurde die Reaktionslösung auf Wasser gegeben, die Niederschlag abgetrennt und getrocknet. Es wurden 35 Teile (entsprechend 89 % der Theorie) 2-Amino-4-cyano-5-diethylamino-thiazol erhalten.
Schmp.: 185 °C

Analog Beispiel 10 wurden die Verbindungen der Beispiele 11 bis 17 hergestellt.

Beispiel 11

4-Cyano-2,5-diamino-thiazol

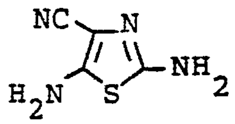

Schmp.: 240 °C

Beispiel 12

2-Amino-4-cyano-5-di-n-propylamino-thiazol

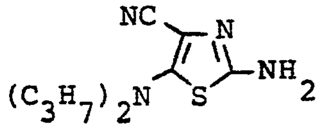

Schmp.: 224 °C

Beispiel 13

2-Amino-4-cyano-5-diisopropylamino-thiazol

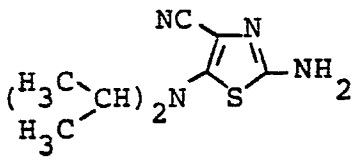

Schmp.: 224 °C

Beispiel 14

2-Amino-5-allylamino-4-cyano-thiazol

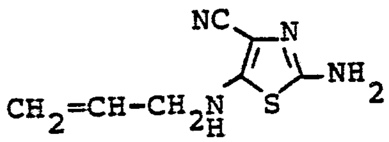

Schmp.: 137 °C

Beispiel 15

2-Amino-4-cyano-5-diallylamino-thiazol

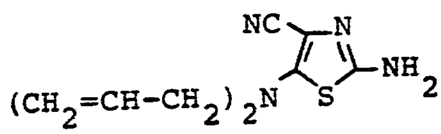

Schmp.: 220 °C

Beispiel 16

2-Amino-4-cyano-5-di-n-butylamino-thiazol

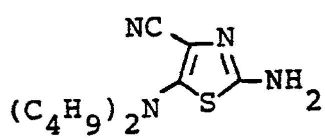

Schmp.: 82 °C

Beispiel 17

2-Amino-4-cyano-5-morpholin-1-yl-thiazol

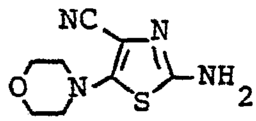

Schmp.: 202 °C

Beispiel 18

2-Amino-4-cyano-5-phenylthio-thiazol

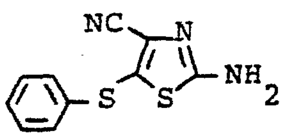

26,4 Teile Thiophenolnatrium wurden in 200 Teilen Methylglykol mit 31,9 Teilen 2-Amino-5-chlor-4-cyano-thiazol auf 80 °C erwärmt. Die Reaktionsmischung wurde auf Wasser gegeben, der Niederschlag abgetrennt und getrocknet. Es wurden 42 Teile (entsprechend 80 % der Theorie) 2-Amino-4-cyano-5-phenylthiothiazol erhalten.

Schmp.: 191 °C

Analog Beispiel 18 wurden die Verbindungen der Beispiele 19 und 20 hergestellt.

Beispiel 19

2-Amino-5-benzthiazol-2-yl-thio-4-cyano-thiazol

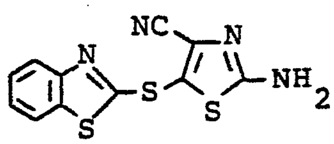

Schmp.: 206 °C

Beispiel 20

2-Amino-4-cyano-5-phenoxi-thiazol

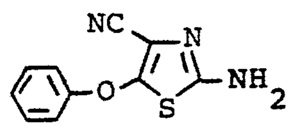

Schmp.: 154 °C

Beispiel 21

2-Amino-4-cyano-5-phenylsulfonyl-thiazol

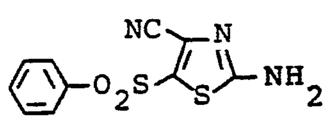

23,3 Teile 2-Amino-4-cyano-5-phenylthio-thiazol wurden in 300 Teilen Essigsäure mit 22,4 Teilen 30 %iger wässriger Wasserstoffperoxidlösung 30 Minuten auf 100 °C erwärmt. Die Reaktionslösung wurde auf Wasser gegeben, der Niederschlag abgesaugt und aus Ethanol umkristallisiert. Es wurden 21 Teile (entsprechend 79 % der Theorie) 2-Amino-4-cyano-5-phenylsulfonyl-thiazol erhalten.

Schmp.: 170 °C

Patentansprüche

1. Verbindungen der allgemeinen Formel I

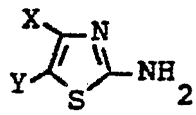

I

in der

X Formyl, Carboxyl oder ein Derivat einer Carboxylgruppe und

Y Wasserstoff, Fluor, Chlor, Brom, Nitro, Rhodan, Alkyl- oder Arylsulfonyl oder gegebenenfalls substituiertes Amino, Hydroxy oder Mercapto sind.

2. Verbindungen gemäß Anspruch 1 der Formel

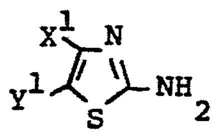

Ia,

in der

$X^1$ CHO, $CH(CO_2CH_3)_2$ oder CN und

$Y^1$ Chlor oder gegebenenfalls substituiertes Amino, Phenylmercapto oder Phenylsulfonyl sind.

3. Verwendung der Verbindungen gemäß Anspruch 1 als Diazokomponenten.

Europäisches Patentamt

# EUROPÄISCHER RECHERCHENBERICHT

0160818
Nummer der Anmeldung

EP 85 10 3386

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | CHEMICAL ABSTRACTS, Band 74, Nr. 25, 21. Juni 1971, Columbus, Ohio, USA; A.P. MOMSENKO et al. "New 2-aminothiazole-4-carboxylic acid derivatives", Seite 576, Zusamenfassung Nr. 141613s; & Nauch Tr., Kursk. Politekh. Inst., Nr. 1, Teil 1, 1968, Seiten 162-164<br>--- | 1,3 | C 07 D 277/56<br>C 07 D 277/40 |
| X | CHEMICAL ABSTRACTS, Band 53, Nr. 19, 10. Oktober 1959, Columbus, Ohio, USA; J.B. DICKEY et al. "Azo dyes from substituted 2-aminothiazoles", Spalten 18004g - 18006e & Journal of Organic Chemistry, Band 24, 1959, Seiten 187-196<br>* Spalten 18004g, h, 18005g* in Verbindung mit CHEMICAL ABSTRATCS, Sixth Collective Index, vol. 51-55, 1957-1961, Subjects; Columbus, Ohio, USA; Seite 11619 s,Spalte 2, Zeile 92, Spalte 3, Zeilen 6,7<br>--- | 1,3 | RECHERCHIERTE SACHGEBIETE (Int. Cl.4)<br>C 07 D 277/00 |
| X | CHEMICAL ABSTRACTS, Band 51, Nr. 20, 25. Oktober 1957, Columbus, Ohio, USA; A. CANAS-RODRIGUEZ "2-Amino-4-polyhydroxyalkylthiazole", Spalte 15499f; & Chemistry and Industry, 1957, Seite 666<br>--- -/- | 1 | |

Der vorliegende Recherchenbericht wurde für alle Patentansprüche erstellt.

| Recherchenort | Abschlußdatum der Recherche | Prüfer |
|---|---|---|
| BERLIN | 01-07-1985 | HASS C V F |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPA Form 1503. 03.82

Europäisches Patentamt

# EUROPÄISCHER RECHERCHENBERICHT

0160818
Nummer der Anmeldung

EP 85 10 3386

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| X | FR-A- 959 908 (SOCIETE GENERALE D'APPLICATIONS THERAPEUTIQUES "THERAPLIX") * Beispiel 3 * --- | 1 | |
| X | CHEMICAL ABSTRACTS, Band 75, Nr. 7, 16. August 1971, Columbus, Ohio, USA; P.P. DIDENKO "Testing of some organic compounds (heterocyclic, aromatic) as anthelmintics in experimental animals", Seite 260, Zusammenfassung Nr. 47793z; & Tr. Vses. Inst. Gel'mintol., Nr. 15, 1969, Seiten 103-108 --- | 1 | |
| X | DE-A-2 500 490 (C M INDUSTRIES) * Ansprüche 1,2; Beispiel 1 * --- | 1 | RECHERCHIERTE SACHGEBIETE (Int. Cl.4) |
| P,X | FR-A-2 540 873 (TOYAMA CHEMICAL CO., LTD.) * Beispiel 4 * --- | 1 | |
| A | US-A-4 324 899 (M.D. FRISHBERG) * Zusammenfassung * --- -/- | 1,3 | |

Der vorliegende Recherchenbericht wurde für alle Patentansprüche erstellt

| Recherchenort | Abschlußdatum der Recherche | Prüfer |
|---|---|---|
| BERLIN | 01-07-1985 | HASS C V F |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

Europäisches Patentamt

**EUROPÄISCHER RECHERCHENBERICHT**

0160818

Nummer der Anmeldung

EP 85 10 3386

Seite 3

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl.4) |
|---|---|---|---|
| A | CHEMICAL ABSTRACTS, Band 60, Nr. 5, 2. März 1964, Columbus, Ohio, USA; C.S. MAHAJANSHETTI et al. "2-Aminothiazole Derivatives.III", Spalten 5474h-5475b; & Journal of the Indian Chemical Society, Band 40, Nr. 11, 1963, Seiten 921-924 | 1,2 | |
| | --- | | |
| A | GB-A- 851 564 (SANDOZ LTD.) * Seite 3, Zeilen 19-28 * | 1 | |
| | ----- | | |
| | | | RECHERCHIERTE SACHGEBIETE (Int. Cl.4) |

Der vorliegende Recherchenbericht wurde für alle Patentansprüche erstellt.

| Recherchenort | Abschlußdatum der Recherche | Prüfer |
|---|---|---|
| BERLIN | 01-07-1985 | HASS C V F |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03.82